# EUROPÄISCHE PATENTSCHRIFT

(11) **EP 3 177 223 B1**
(45) Veröffentlichungstag und Bekanntmachung des Hinweises auf die Patenterteilung: **16.10.2019**
(21) Anmeldenummer: 15753326.6
(22) Anmeldetag: 03.08.2015
(51) Int. Cl.: A61B 17/86, A61B 17/88

(54) **SCHRAUBE MIT EINBRINGPFOSTEN**
SCREW WITH INSERTION POST
VIS COMPRENANT UN PORTE-IMPLANT

(30) Priorität: 05.08.2014 DE 202014006372 U
(43) Veröffentlichungstag der Anmeldung: 14.06.2017
(73) Patentinhaber: Medartis Holding AG, 4057 Basel (CH)
(72) Erfinder: LANGER, Barry, 4106 Therwil (CH); SCHEUBLE, Peter, 79418 Schliengen (DE); AMMANN, Marc, 4148 Pfeffingen (CH); POLZHOFER, Herbert, 4053 Basel (DE)
(74) Vertreter: Hepp Wenger Ryffel AG
(86) Internationale Anmeldenummer: PCT/EP2015/067837
(87) Internationale Veröffentlichungsnummer: WO 2016/020329

(56) Entgegenhaltungen:
- FR-A1- 2 768 781
- FR-A1- 2 781 998

## Beschreibung

Die Erfindung betrifft Schrauben mit einem Einbringpfosten sowie Verfahren zur Herstellung von Schrauben mit einem Einbringpfosten gemäss dem Oberbegriff der unabhängigen Ansprüche.

Nachfolgend wird die Erfindung der Einfachheit halber anhand von Knochenschrauben beschrieben. Dies soll aber auf keinen Fall einschränkend verstanden werden. Die erfindungsgemässen Schrauben können auch in anderen Gebieten, in welchen Schrauben benötigt werden, zum Einsatz kommen.

Schrauben kommen zum Einsatz, wo Einzelteile aneinander fixiert werden sollen. So werden beispielsweise Knochenteile nach einem Bruch mit Knochenschrauben fixiert. Oftmals kommen dabei auch Knochenplatten zum Einsatz, welche mit Hilfe von Schrauben an den Knochen fixiert werden.

Beim Eindrehen von Schrauben besteht die Gefahr, dass die Schraube so fest eingedreht wird, dass Teile der Schraube oder des Knochens beschädigt werden. Dadurch kann es vorkommen, dass die Funktion der Schraube nicht mehr gewährleistet ist. Zudem kann der Antrieb beschädigt werden, wodurch ein Entfernen der Schraube durch Ausdrehung nicht mehr sichergestellt werden kann. Dies kann gerade auch bei einer maschinellen Eindrehung geschehen, bei welcher die Drehmomentzunahme weniger bzw. nicht vom Benutzer bemerkt wird.

FR 2 768 781 A1 beschreibt eine Schraube mit einem Einbringpfosten.

Die WO 97/27812 schlägt vor, eine Knochenschraube bereitzustellen, welche mit einem Eindrehwerkzeug als Einheit vorliegt. Das Eindrehwerkzeug besitzt einen Schaft, welcher mit einem Schraubenkopf verbunden ist. Im Anschlussbereich zwischen dem Kopf und dem Schaft ist eine Sollbruchstelle ausgebildet. Diese Einheit wird in den Knochen eingeschraubt. Während dem Einschrauben wächst das auf die Sollbruchstelle wirkende Drehmoment. Wenn die Einschraubtiefe erreicht ist, ist das Drehmoment so gross, dass der Schraubenkörper vom Schaft an der Sollbruchstelle abbricht.

Ein Teil des Eindrehwerkzeugs verbleibt mit dem Schraubenkopf verbunden. Die Bruchstelle kann dabei rau sein und umliegendes Gewebe reizen oder beschädigen. Zudem verhindert die Bruchkontur, dass ein herkömmliches Ausdrehwerkzeug in den Schraubenkopf eingeführt werden kann, wenn die Schraube wieder gelöst werden soll. Um die Schraube zu entfernen wird daher ein Spezialwerkzeug benötigt. Solche Spezialwerkzeuge sind zum einen sehr teuer, zum anderen sind sie bei den auf Explantationen spezialisierten Ärzten nicht immer vorhanden.

Es ist daher die Aufgabe der vorliegenden Erfindung, eine Schraube bereitzustellen, welche die Nachteile des bekannten Standes der Technik verhindert. Insbesondere ist es die Aufgabe der vorliegenden Erfindung eine Schraube bereitzustellen, bei welcher die Bruchstelle das umliegende Gewebe nicht beschädigen kann und welche einfach wieder lösbar ist.

Erfindungsgemäss wird das Problem gemäss den Merkmalen der unabhängigen Ansprüche gelöst. Dabei wird eine Schraube, insbesondere eine Knochenschraube, mit einem Kopf, einem Schaft und einer Spitze vorgeschlagen. Die Schraube weist auf der Kopfseite eine Ausnehmung mit einer Kontur auf. Eine Gegenkontur eines Werkzeugs ist mit der Kontur der Ausnehmung in Wirkverbindung bringbar. Die Schraube ist ausserdem in der Ausnehmung mit einem Einbringpfosten versehen. Dabei ist der Einbringpfosten über eine Sollbruchstelle mit der Knochenschraube verbunden. Die Sollbruchstelle ist innerhalb der Ausnehmung so angeordnet, dass nach Bruch und Entfernung des Einbringpfostens eine Drehmomentübertragung zwischen dem Werkzeug und der Schraube über die Kontur und Gegenkontur möglich ist.

Die Schraubenspitze beschreibt allgemein das entgegengesetzte Ende der Kopfseite der Schraube. Die Schraubenspitze muss dabei nicht zwingend spitz ausgebildet sein, sondern kann z.B. auch abgerundet oder abgeflacht ausgebildet sein.

Der Kopf der Schraube beschreibt allgemein ein Endstück der Schraube, welches die Ausnehmung aufweist. Der Schraubenkopf muss dabei nicht zwingend als Querschnittserweiterung gegenüber dem Schaft ausgebildet sein. Der Schaft und der Kopf können auch denselben oder sogar einen kleineren Querschnitt aufweisen.

Der Schaft der Schraube ist dabei zumindest teilweise mit einem Gewinde versehen. Eine Steigung des Gewindes ist je nach Funktion der Schraube wählbar. Es ist auch möglich, dass die Schraube ein Gewinde mit mehreren Steigungen aufweist. Das Gewinde verläuft vorzugsweise bis in die Spitze. Das Gewinde kann selbstbohrend oder nicht selbstbohrend ausgebildet sein.

Die Kontur der Ausnehmung ist so ausgebildet, dass sie eine Momentübertragung mit der Gegenkontur des Werkzeuges ermöglicht. Vorzugsweise ist die Kontur dabei als Innenkontur und die Gegenkontur als Aussenkontur ausgebildet.

Alternativ ist denkbar, dass die Kontur als Aussenkontur ausbildet ist. Dabei ist die Kontur beispielsweise als Stift in der Ausnehmung ausgebildet. Die Gegenkontur kann bei dieser Ausbildung als Innenkontur ausgebildet sein, welche über den Stift gestülpt wird.

Innerhalb der Ausnehmung ist die Schraube über eine Sollbruchstelle mit einem Einbringpfosten verbunden. Die Verbindung ist dabei über die Sollbruchstelle indirekt ausgebildet.

Als direkte Verbindung wird verstanden, wenn die Sollbruchstelle das einzige Verbindungsstück zwischen Einbringpfosten und Schraube darstellt.

Als indirekte Verbindung wird verstanden, wenn die Verbindung erst über eine Pfostenspitze zustande kommt.

Als Pfostenspitze wird vorliegend ein Endstück des Einbringpfostens verstanden, welches durch ein Brechen der Sollbruchstelle vom restlichen Einbringpfosten getrennt werden kann.

Die vorgeschlagene Schraube kann über den Einbringpfosten eingedreht werden. Während des Eindrehens nimmt das Drehmoment zu. Sobald ein gewisses Drehmoment erreicht ist, bricht der Einbringpfosten an der Sollbruchstelle. Dadurch wird zumindest ein Teil des Einbringpfostens, welcher von der Sollbruchstelle in Richtung Kopfseite zeigt, von der Schraube gelöst.

Vorzugsweise erfolgt der Bruch der Sollbruchstelle, bevor die Endstellung der Schraube erreicht wird. Über die Kontur der Ausnehmung kann die Schraube danach mit dem Werkzeug in die Endposition gedreht werden.

Alternativ ist die Einheit Einbringpfosten-Schraube so konzipiert, dass die Sollbruchstelle bricht, sobald die Schraube ihre Endposition eingenommen hat.

Die Sollbruchstelle verhindert dabei, dass ein zu grosses Drehmoment entsteht. Indem das Drehmoment nicht zu gross werden kann, wird verhindert, dass der Knochen und/oder die Schraube (z.B. die Kontur der Schraube) oder eine allfällige Platte, welche mit der Schraube befestigt wird, beschädigt wird. Dadurch wird sichergestellt, dass die Schraube ihre Fixierungsaufgabe wahrnehmen kann. Des Weiteren wird mit einer unbeschädigten Kontur sichergestellt, dass die Schraube einfach wieder lösbar ist, sobald die Fixierung nicht mehr benötigt wird.

Alternativ kann die Sollbruchstelle durch Abbrechen (Biegemoment) statt Abdrehen (Torsionsmoment) gebrochen werden. Dies geschieht vorzugsweise, bevor eine Endstellung der Schraube erreicht wird. Die Endstellung kann danach wiederum über die Kontur der Ausnehmung und die Gegenkontur des Werkzeugs erreicht werden.

Das Werkzeug kann, wie vorgängig beschrieben, dazu dienen, die Schraube in die Endposition zu bringen. Das Werkzeug dient aber auch dazu, die Schraube wieder zu lösen, z.B. wenn die Fixierung nicht mehr benötigt wird. Dazu ist eine Drehmomentübertragung zwischen der Gegenkontur des Werkzeugs und der Kontur der Ausnehmung nötig. Die Sollbruchstelle zwischen Schraube und Einbringpfosten ist so versenkt innerhalb der Ausnehmung angeordnet, dass nach dem Bruch an der Sollbruchstelle und einer Entfernung des abgebrochenen Endes des Einbringpfostens eine Drehmomentübertragung zwischen der Kontur der Ausnehmung und der Gegenkontur des Werkzeugs möglich ist.

Die Sollbruchstelle ist daher versenkt gegenüber der Kopfseite der Schraube angeordnet, insbesondere ist die Sollbruchstelle um mehr als 50 % einer Länge der Ausnehmung versenkt angeordnet, besonders bevorzugt ist die Sollbruchstelle ungefähr auf einem Niveau einer Grundfläche der Ausnehmung, bevorzugt genau auf dem Niveau der Grundfläche der Ausnehmung, angeordnet. Die Sollbruchstelle ist dabei innerhalb der Schraube angeordnet. Dadurch wird umliegendes Gewebe nach Brechen der Sollbruchstelle nicht durch die Bruchstelle irritiert, wodurch Reizung und/oder Beschädigung des Gewebes verhindert wird. Ausserdem ist die Kontur für das Werkzeug zugänglich.

Bevorzugt ist die Sollbruchstelle auf der der Schraubenspitze zugeordneten Seite der Kontur angeordnet. Eine derartige Anordnung hat zur Folge, dass die gesamte Kontur nach Bruch der Sollbruchstelle und Entfernung des Einbringpfostens nicht von der Sollbruchstelle und/oder der allfälligen Pfostenspitze bedeckt ist. Dadurch ist auch die gesamte Kontur für die Gegenkontur des Werkzeugs frei zugänglich. Dies ermöglicht eine gute Drehmomentübertragung zwischen Werkzeug und Schraube. Weiter ist die Sollbruchstelle bei einer derartigen Anordnung von der Kopfseite der Schraube mindestens über die gesamte Länge der Kontur getrennt. Die Sollbruchstelle ist dadurch in keinerlei Kontakt mit dem umliegenden Gewebe.

Bevorzugt weist die Ausnehmung anschliessend an die Kontur gegen die Schraubenspitze hin eine Öffnung für eine Montage des Einbringpfostens auf. Die Pfostenspitze kann dabei in der Öffnung montiert werden. Die Öffnung ist bevorzugt mit kleinerem Durchmesser als die Ausnehmung mit der Kontur ausgebildet. Die Öffnung weist bevorzugt eine andere Kontur als die Kontur der Ausnehmung auf. Die Sollbruchstelle ist dabei bevorzugt am Übergang zwischen der Öffnung zur Montage des Einbringpfostens und der Kontur, d.h. ungefähr auf dem Niveau der Grundfläche der Ausnehmung, angeordnet.

Die Sollbruchstelle kann auch innerhalb der Öffnung, d.h. unter dem Niveau der Grundfläche der Ausnehmung, angeordnet sein.

Alternativ ist die Sollbruchstelle am unteren Ende der Ausnehmung mit Kontur ausgebildet. Dadurch ist die Kontur immer noch zu grossen Teilen unbedeckt und eine Drehmomentübertragung zwischen Werkzeug und Schraube immer noch gut möglich.

Im Zusammenhang mit der vorliegenden Erfindung sind auch kanülierte Schrauben denkbar. Dabei ist ein Durchgangsloch in der Schraube ausgebildet. Kanülierte Schrauben sind sowohl für Schrauben mit oder ohne Öffnung zur Montage des Einbringpfostens denkbar. Die Durchgangsöffnung besitzt bevorzugt einen kleineren Durchmesser als die Ausnehmung und die Öffnung zur Montage des Einbringpfostens. Alternativ ist die Öffnung zur Montage Teil der Durchgangsöffnung. Durch die Kanülierung kann die Schraube beispielsweise über einen zuvor im Knochen gesetzten Draht (z.B. K-wire) eingebracht werden.

Bevorzugt ist die Sollbruchstelle als Materialschwächung mit reduziertem Torsionsbruch- und Biegebruchmoments, bevorzugt in Form einer Einschnürung, ausgebildet. Die Sollbruchstelle ist dadurch schwächer ausgebildet als der Einbringpfosten, zumindest an einem in Richtung Kopfseite zeigenden und an die Sollbruchstelle anliegenden Abschnitt. Vorzugsweise ist die Sollbruchstelle schwächer als der gesamte restliche Einbringpfosten. Über Dimension und/oder Form und/oder Material der Sollbruchstelle kann bestimmt werden, bei welchem Drehmoment die Sollbruchstelle bricht.

Die Sollbruchstelle ist vorzugsweise aus demselben Material, vorzugsweise Titan, Titanlegierung, Implantatstahl, resorbierbarem metallischen oder nichtmetallischen Material, wie die umgebenden Stellen des Einbringpfostens ausgebildet.

Die Sollbruchstelle kann beispielsweise durch einen spanabhebenden Prozess zustande kommen.

Alternativ kann die Sollbruchstelle auch aus einem anderen Material ausgebildet sein. So ist beispielsweise denkbar, dass der Einbringpfosten bis auf die Sollbruchstelle weitgehend aus Metall gefertigt ist, die Sollbruchstelle jedoch aus Kunststoff, einem Klebstoff, einem anderen Metall oder einem anderweitigen Material, welches ein reduziertes Torsionsbruch- und/oder Biegebruchmoment aufweist, besteht. Die Sollbruchstelle kann in diesem Fall auch ohne Einschnürung ausgebildet sein.

Der Einbringpfosten ist einteilig über die Sollbruchstelle mit einer Pfostenspitze versehen. Die Pfostenspitze lässt sich dabei bevorzugt in der Öffnung zur Montage des Einbringpfostens montieren.

Einteilig ausgebildet heisst, dass der Einbringpfosten mit Sollbruchstelle und Pfostenspitze aus einem Stück angefertigt ist. Die Pfostenspitze kann so in der Ausnehmung, bevorzugt in der Öffnung, montiert werden, dass ein Drehmoment zum Eindrehen der Schraube über die Pfostenspitze auf die Schraube übertragbar ist. Dabei weist die Pfostenspitze bevorzugt eine Pfostenspitzenkontur zur Drehmomentsübertragung auf eine Pfostenspitzengegenkontur auf. Die Pfostenspitze verbleibt nach dem Bruch vorzugsweise in der Schraube.

Eine einteilige Ausbildung ist einfach herzustellen, da keine Teile zusammengefügt werden müssen. Die Pfostenspitzenkontur und die Sollbruchstelle werden vorzugsweise in den Einbringpfosten eingearbeitet.

Vorzugsweise ist die Sollbruchstelle so ausgelegt, dass sie bei einem Abdrehmoment bricht, welches kleiner oder gleich dem üblichen Anzugsmoment dieser Schraube ist.

Typischerweise wird bei medizinischen Anwendungen beim Eindrehvorgang des Schaftes der Schraube dieses Abdrehmoment noch nicht erreicht. Es wird üblicherweise erreicht, sobald die Schraube ihre Endposition erreicht hat, also in der Regel wenn ein Kopf oder ein Endstück der Schraube auf eine Knochenplatte oder den Knochen trifft. Bei diesem Moment werden der Knochen und/oder die Schraube noch nicht beschädigt. Die Sollbruchstelle ist daher so konzipiert, dass zum einen eine Endstellung der Schraube durch Eindrehen mit dem Einbringpfosten erreicht wird, zum anderen aber keine Schäden am Knochen und/oder an der Schraube auftreten.

Alternativ sind je nach Anwendung höhere oder tiefere Drehmomente denkbar.

In einer bevorzugten Ausführungsform ist der Einbringpfosten mit der Pfostenspitze in die Öffnung zur Montage des Einbringpfostens eingepresst.

Dabei wird der Einbringpfosten vorzugsweise mit einteiliger Pfostenspitze in die Öffnung gepresst. Über die Pfostenspitze ist eine Momentübertragung vom Einbringpfosten auf die Schraube möglich.

Durch das Einpressen kann eine stabile Verbindung hergestellt werden, welche keine zusätzlichen Verbindungselemente benötigt.

In einer weiteren alternativen, bevorzugten Ausführungsform wird der Einbringpfosten in die Öffnung eingeschraubt. Dabei ist die Pfostenspitze zumindest teilweise mit einem Gewinde versehen, welches in ein Gegengewinde der Öffnung verschraubt werden kann. Dadurch ist die Pfostenspitze nach dem Einschrauben in der Öffnung angeordnet. Im Falle einer kanülierten Schraube kann diese Öffnung jedoch als Durchgangsloch mit zumindest einem teilweisen Gewinde ausgebildet sein.

Eine Schraubverbindung stellt eine stabile, lösbare Verbindung dar, welche keine zusätzlichen Verbindungselemente benötigt. Die Sollbruchstelle ist bevorzugt direkt anschliessend an das Gewinde der Pfostenspitze ausgebildet. Das Anzugsmoment für die Gewindeverbindung wird hierbei so hoch gewählt, dass es höher als das Ein- bzw. Ausdrehmoment für den Schraubenschaft jedoch kleiner als das Abdrehmoment der Sollbruchstelle ist. Somit ist ein Lösen der Schraube ohne Zerstörung der Sollbruchstelle während der Implantation möglich, z.B. wenn festgestellt wird, dass die Schraube noch nicht an der optimalen Stelle sitzt. Für eine Erhöhung des Anzugsmoments wird eine zur Schraubverbindung zusätzliche Pressverbindung verwendet.

Dabei wird der eingeschraubte Einbringpfosten vorzugsweise zusätzlich von aussen verpresst.

Es kann auch eine Kombination der vorstehend beschriebenen Verbindungsmöglichkeiten verwendet werden.

Bevorzugt ist die Kontur der Ausnehmung als Schlitz, Kreuzschlitz, Innenmehrkant, Innenvielrund oder Phillipps, besonders bevorzugt als Sechskant oder Innensechsrund ("Torx"), d.h. als standardisierte oder genormte Kontur, ausgebildet. Sowohl Sechskant als auch Innensechsrund bilden zuverlässige Möglichkeiten der Momentübertragung.

Mit diesen bevorzugten Konturen wird kein Spezialwerkzeug benötigt, um die Schraube wieder zu lösen oder nach dem Bruch der Sollbruchstelle fester anzuziehen. Da Spezialwerkzeuge meist teuer in der Herstellung -und im Ankauf- sind, ist eine Lösung mit Konturen wie Sechskant oder Innensechsrund kostengünstiger. Zudem sind Spezialwerkzeuge nicht immer vorhanden und das Anziehen, und vor allem auch das Ausdrehen bei der Explantation in einer Klinik/Praxis, die nicht über die entsprechenden Werkzeuge verfügt wäre in diesem Fall nicht bzw. nur schwer möglich.

Vorzugsweise weist der Einbringpfosten zusätzlich eine Einbringkontur zur Momentenübertragung einer handelsüblichen, (medizinischen) Bohrmaschine auf.

Die Einbringkontur kann auch als Griff, wie beispielsweise von Schraubendrehern bekannt, ausgebildet sein.

Alternativ ist keine spezielle zusätzliche Kontur ausgebildet und die Momentübertragung auf den Einbringpfosten findet ohne zusätzliche Kontur z.B. rein reibschlüssig über das Backenfutter einer Bohrmaschine statt.

Bevorzugt bestehen die verschiedenen Teile des Einbringpfostens und der Schraube aus Titan, Titanlegierung, Implantatstahl, resorbierbarem metallischem oder nichtmetallischem Material.

Insbesondere die Pfostenspitze kann aus resorbierbarem Material bestehen. Die Pfostenspitze verbleibt nach dem Bruch der Sollbruchstelle vorzugsweise in der Schraube. Falls die Schraube als Knochenschraube verwendet wird, bleibt die Pfostenspitze daher im Körper. Falls die Pfostenspitze die Kontur der Ausnehmung noch teilweise bedeckt, wird durch die Resorption die ganze Kontur wieder frei. Dies führt dazu, dass das Werkzeug mit der Gegenkontur zur Entfernung der Schraube bei der Explantation besser mit der Kontur in Wirkverbindung treten kann. Dadurch wird ein Lösen der Schraube erleichtert.

Bevorzugt enthält der Einbringpfosten eine produktspezifische Kennzeichnung, vorzugsweise eine Lotnr, einen Barcode oder ein Logo. Die produktspezifisch Kennzeichnung kann nach dem Bruch der Sollbruchstelle ausgelesen und/oder aufbewahrt werden.

Durch die Kennzeichnung ist die implantierte Schraube in Kenntnis des abgetrennten Einbringpfostens im Nachhinein identifizierbar.

Ein weiterer Aspekt der Erfindung betrifft ein Set mit mindestens einer erfindungsgemässen Schraube und einem Werkzeug, das eine zur Kontur der Ausnehmung komplementäre Gegenkontur der Schraube aufweist.
Ein weiterer Aspekt der Erfindung betrifft ein Set mit wenigstens einer Knochenplatte und wenigstens einer erfindungsgemässen Schraube wie vorgängig beschrieben.

Die Schraube(n) dient dabei vorzugsweise der Fixierung der Knochenplatte(n) am Knochen. Die Schraube(n) und die Knochenplatte(n) können dabei eine Verblockungskontur oder ein Gewinde aufweisen, wodurch die Schraube in der Platte verblockt werden kann. Details der Verblockung sind z.B. in der EP 1 608 278 A1 beschrieben, deren Inhalt hier durch Referenz aufgenommen wird.

Vorzugsweise umfasst dieses Set zusätzlich mindestens ein Werkzeug mit einer zur Kontur der Ausnehmung komplementären Gegenkontur. Mit dem Werkzeug kann die Schraube beispielsweise nach dem Abbrechen des Einbringpfostens noch fester angezogen oder/und nach Verheilung eines Knochenbruchs wieder gelöst und entfernt werden.

Des Weiteren betrifft die Erfindung ein Verfahren zur Herstellung einer Schraube mit einem Einbringpfosten gemäß Anspruch 14. Es umfasst folgende Schritte:
- Bereitstellen eines Schraubenkörpers mit einem Schraubenkopf, Schaft und Schraubenspitze, wobei der Schraubenkörper eine Ausnehmung mit einer Kontur und anschliessender Öffnung zur Aufnahme des Einbringpfostens aufweist,
- Bereitstellung eines Einbringpfostens, welcher über eine Sollbruchstelle mit einer Pfostenspitze verbunden ist
- Zusammenfügen des Einbringpfostens und des Schraubenkörpers über die Pfostenspitze und die Öffnung zur Aufnahme des Einbringpfostens.

In einem bevorzugten Verfahren wird der Einbringpfosten in den Schraubenkörper eingeschraubt. Bevorzugt ist der Einbringpfosten zusätzlich von aussen mit dem Schraubenkörper verpresst.

Alternativ kann der Einbringpfosten auch nur in den Schraubenkörper eingepresst werden.

Bevorzugt wird der Einbringpfosten mit einer Kombination der beschriebenen Methoden mit dem Schraubenkörper verbunden.

Weitere vorteilhafte Ausgestaltungen der Erfindung ergeben sich aus der nachfolgenden Beschreibung der Ausführungsbeispiele in Kombination mit den schematischen Figuren. Es zeigen schematisch:
- Figuren 1a/b:: Einen Längsschnitt durch eine erste Ausführungsform einer erfindungsgemässe Schraube.
- Figur 2:: Einen Längsschnitt durch eine alternative Schraube.
- Figur 3:: Einen Längsschnitt durch eine weitere Ausführungsform einer erfindungsgemässen Schraube.
- Figur 4:: Einen Längsschnitt durch eine weitere Schraube.
- Figuren 5a/b/c:: Detailansichten von erfindungsgemässen Einbringpfosten mit unterschiedlichen Sollbruchstellen.
- Figur 6:: eine erfindungsgemässe Schraube nach dem Abdrehen/Abbrechen des Einbringpfostens mit Werkzeug.
- Figur 7:: einen Längsschnitt durch eine erste Ausführungsform einer erfindungsgemässen Schraube nach dem Abdrehen/Abbrechen des Einbringpfostens mit Werkzeug.
- Figur 8:: einen Längsschnitt durch eine alternative Ausführungsform einer erfindungsgemässen Schraube nach dem Abdrehen/Abbrechen des Einbringpfostens mit Werkzeug.
- Figur 9:: einen Querschnitt durch einen Schraubenkopf einer erfindungsgemässen Schraube mit Einbringpfosten.

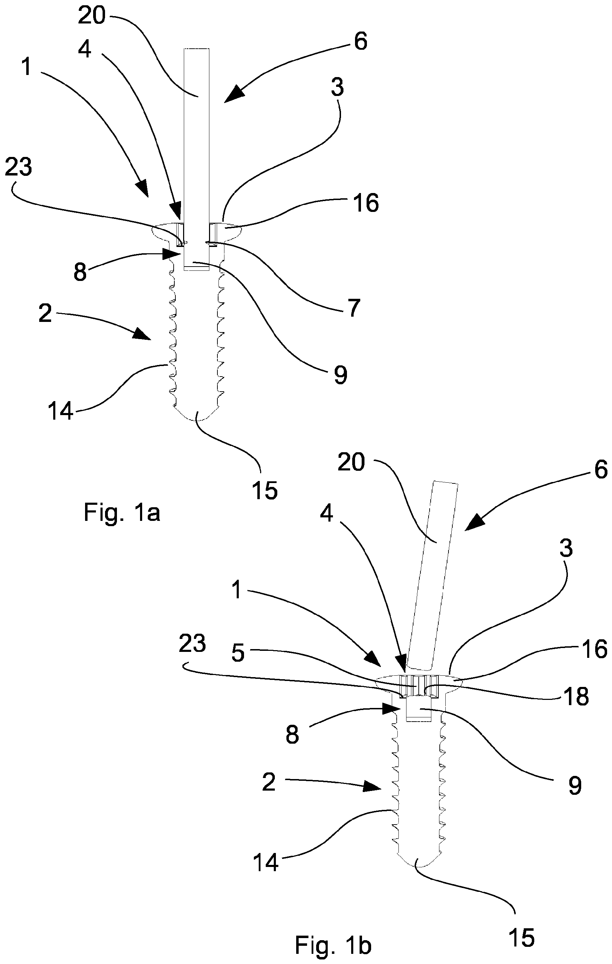

Figuren 1a und 1b zeigen einen Längsschnitt durch eine erste Ausführungsform einer erfindungsgemässen Schraube 1. Die Schraube 1 hat einen Schaft 2, welcher mit einem Gewinde 14 versehen ist. Die Schraube 1 hat zudem eine Spitze 15 und einen der Spitze 15 gegenüberliegenden Schraubenkopf 16. Der Schraubenkopf 16 besitzt eine Kopfseite 3. An der Kopfseite 3ist eine Ausnehmung 4 ausgebildet. Anschliessend an die Ausnehmung 4 ist eine Öffnung 8 zur Aufnahme einer Pfostenspitze 9 eines Einbringpfostens 6 vorhanden. Die Öffnung 8 zur Aufnahme der Pfostenspitze 9 besitzt ein Innengewinde (nicht gezeigt). Die Spitze 9 besitzt ein Aussengewinde (nicht gezeigt), welches in das Innengewinde der Öffnung 8 eingeschraubt werden kann.

In Figur 1a ist die Pfostenspitze 9 über eine Sollbruchstelle 7 mit einem Pfostenschaft 20 des Einbringpfostens 6 verbunden. Der Pfostenschaft 20 ist in den Figuren 1a und 1b nur teilweise dargestellt. Die Sollbruchstelle 7 ist dabei als Einschnürung zwischen der Spitze 9 und dem Pfostenschaft 20 ausgebildet. Der Einbringpfosten 6 ist daher indirekt über die Sollbruchstelle 7 mit der Schraube 1 verbunden. Die indirekte Verbindung von Pfostenspitze und Schraube kann über ein konisches oder zylindrisches Gewinde, durch Verpressen, Verkleben, Schrumpfen, Crimpen oder andere Verbindungsverfahren erfolgen bzw. als Kombination mehrerer dieser Verbindungsverfahren. Bei einer Verbindung, welche z.B. nur durch Verpressen, Verkleben, Schrumpfen oder Crimpen erfolgt, können die Gewinde auch entfallen.

Bei einem Eindrehen der Schraube 1 erhöht sich das Drehmoment. Die Sollbruchstelle 7 ist so ausgelegt, dass sie vor Erreichen des Anzugsmoments bricht. In Figur 1b ist der Pfostenschaft 20 des Einbringpfostens 6 an der Sollbruchstelle 7 von der Pfostenspitze 9 abgebrochen. Die Pfostenspitze 9 weist dadurch eine Bruchstelle 18 auf. Die Bruchstelle 18 befindet sich ungefähr auf dem Niveau der Grundfläche 23 der Ausnehmung 4. Dadurch kommt die Bruchstelle 18 nicht in Kontakt mit umliegendem Gewebe, wodurch eine Reizung dieses Gewebes verhindert wird.

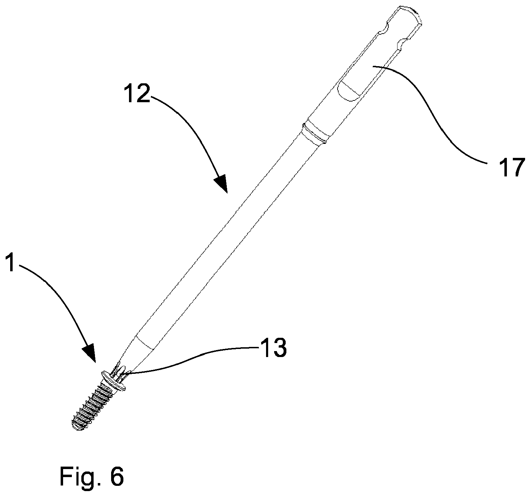

Die Ausnehmung 4 besitzt eine Kontur 5. Die Kontur ist als Innensechsrund ausgebildet. Nach Entfernung des Pfostenschafts 20 des Einbringpfostens 6 liegt die Kontur 5 frei. Ein Werkzeug 12 (siehe z.B. Fig. 6) mit einer passenden Gegenkontur 13 (siehe z.B. Fig. 6) kann mit der Kontur 5 in Wirkverbindung gebracht werden. Mit Hilfe des Werkzeugs 12 kann die Schraube 1 in eine Endposition eingedreht werden. Die Schraube 1 kann mit Hilfe des Werkzeugs 12 auch ausgedreht werden, sobald die Schraube 1 nicht mehr benötigt wird.

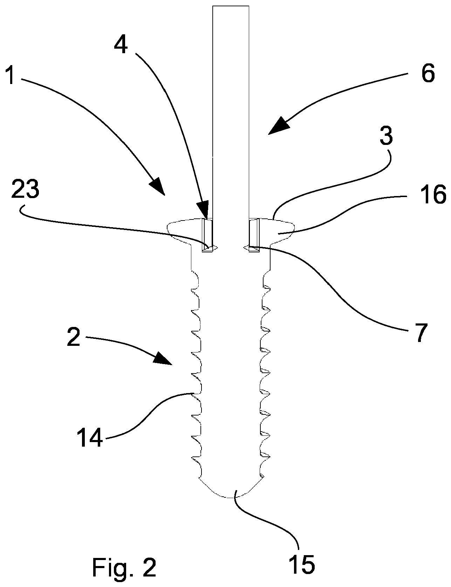

Figur 2 zeigt eine alternative Schraube 1. Der Einbringpfosten 6 und die Schraube 1 sind dabei einstückig ausgebildet. Beide Teile sind aus einem Stück Material angefertigt. Die Sollbruchstelle 7 bildet dabei direkt die Verbindungsstelle zwischen dem Einbringpfosten 6 und der Schraube 1. Nach einem Abbrechen des Einbringpfostens 6 an der Sollbruchstelle 7, wird sich eine Bruchstelle 8 wiederum ungefähr auf dem Niveau der Grundfläche 23 der Ausnehmung 4 befinden (nicht gezeigt). Die Ausnehmung besitzt wiederum eine Kontur 5, über welche mit Hilfe des Werkzeugs 12 (nicht gezeigt) nach Abbrechen der Sollbruchstelle 7 die Schraube 1 weiter eingedreht oder ausgedreht werden kann.

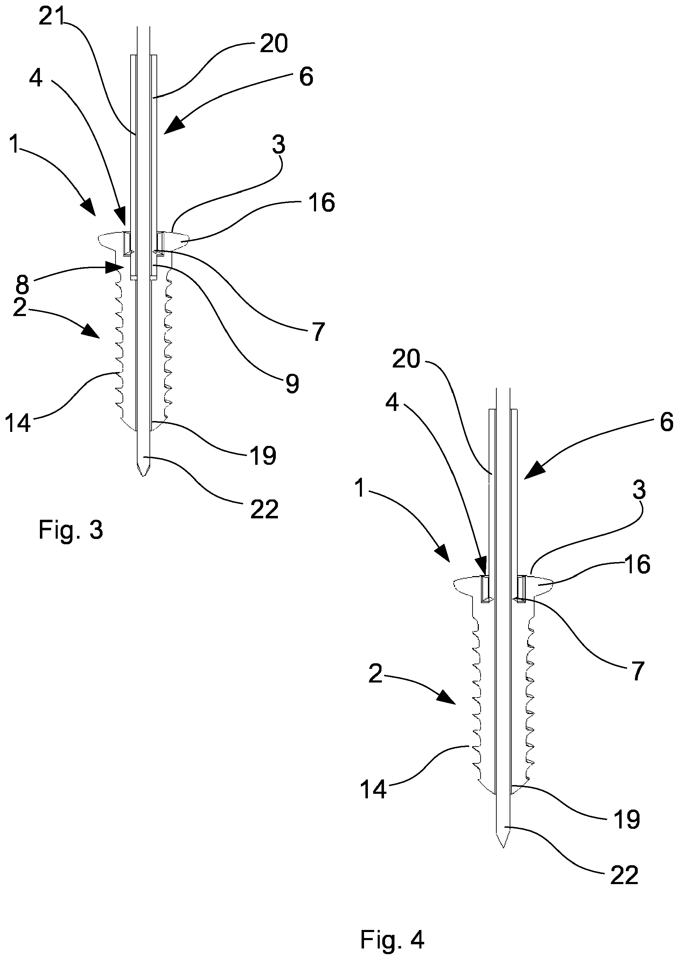

Figur 3 zeigt eine weitere Ausführung einer erfindungsgemässen Schraube 1. Die Schraube 1 und der Einbringpfosten sind wie in der Ausführungsform der Figuren 1a und 1b indirekt über die Sollbruchstelle 7 verbunden. Die Schraube 1 ist in dieser Ausführungsform kanüliert. Die Kanülierung 19 erstreckt sich dabei über den gesamten Schaft 2 der Schraube 1. Auch der Einbringpfosten weist eine Kanülierung 21 auf. Die Kanülierungen 19, 21 sind bei eingeschraubtem Einbringpfosten 6 in Fluidverbindung. Dadurch ist eine durchgängige Kanülierung vorhanden.

Innerhalb der Kanülierungen 19, 21 befindet sich ein Draht 22, ein sogenannter Kirschner- oder K-wire. Solche Drähte 22 werden in verschiedenen orthopädischen und anderen Operationen verwendet. Der Draht 22 wird in den Knochen eingesetzt. Über die Kanülierung 19, 21 ist die Schraube 1 über den Draht 22 in den Knochen einbringbar. Der Draht 22 dient dabei als Führung.

Figur 4 zeigt eine weitere Schraube 1. Wie in Figur 3 ist eine Kanülierung 19 in der Schraube 1 vorhanden. Der Einbringpfosten 6 und die Schraube 1 sind wie in Figur 2 einstückig ausgebildet. Die Kanülierung 19 ist durchgängig durch die Schraube 1 und den Einbringpfosten 6 ausgebildet. Innerhalb der Kanülierung 19 ist wiederum ein Draht 22 gezeigt. Die Schraube 1 wurde wiederum über die Kanülierung 19 und den Draht 22 eingebracht.

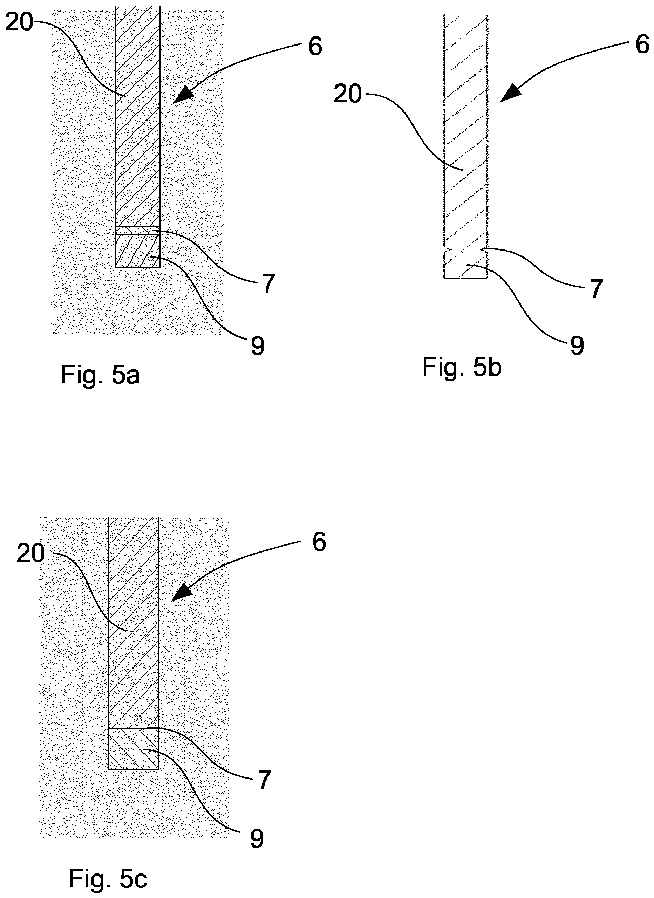

Die Figuren 5a, b, c zeigen Detailansichten von Einbringpfosten 6. Die Einbringpfosten 6 weisen Pfostenspitzen 9 und Pfostenschäfte 20 auf, welche über Sollbruchstellen 7 miteinander verbunden sind.

Bei der in Figur 5a gezeigten Ausführung ist die Sollbruchstelle 7 als Materialvariation realisiert worden. Die Sollbruchstelle 7 ist daher aus einem bruchschwächeren Material als die Pfostenspitze 9 und der Pfostenschaft 20 gebildet. Die Pfostenspitze 9 und der Pfostenschaft 20 sind z.B. aus Titan gefertigt, die Sollbruchstelle z.B. aus einem biokompatiblen Kunststoff, welcher die beiden Teile verklebt. Dadurch wird die Sollbruchstelle 7 bei einem gewissen Drehmoment brechen.

Bei der in Figur 5b gezeigten Ausführung ist die Sollbruchstelle 7 als Einschnürung ausgebildet. Die Einschnürung ist durch Materialabtragung an dieser Stelle zustande gekommen. Die Pfostenspitze 9 und der Pfostenschaft 20 sind einteilig ausgebildet.

Bei der in Figur 5c gezeigten Ausführung ist die Sollbruchstelle durch eine Verbindung mit reduziertem Widerstandsmoment gegen Biegung und Torsion ausgebildet. Solche Verbindungen können beispielsweise Schweissverbindungen sein. Alternative Verbindungen sind Pressverbindungen, Schrumpfverbindungen, Crimpverbindungen, Klebeverbindungen und anderweitige passende Verbindungen, wie auch Kombinationen der Verbindungen.

Figur 6 zeigt eine erfindungsgemässe Schraube 1 nach Entfernung des Pfostenschafts 20 des Einbringpfostens 6 (nicht gezeigt). In die Ausnehmung 4 (in Fig. 6 nicht sichtbar) ist ein Werkzeug 12 eingeführt. Dabei ist eine Gegenkontur 13 des Werkzeugs 12 in Wirkverbindung mit der Kontur 5 (in Fig. 6 nicht sichtbar) der Ausnehmung 4 (in Fig. 6 nicht sichtbar). Die Schraube 1 kann durch das Werkzeug 12 noch weiter eingedreht werden. Sobald die Schraube nicht mehr benötigt wird oder explantiert werden muss, kann die Schraube mit Hilfe des Werkzeugs 12 gelöst werden.

Die Kontur 5 (nicht sichtbar) der Ausnehmung ist z.B. als Innensechsrund ausgebildet. Diese gängige Kontur stellt sicher, dass die Schraube 1 mit einem standardisierten/genormten Werkzeug 12 weiter eingedreht bzw. ausgedreht werden kann.

Das Werkzeug 12 besitzt an einem der Gegenkontur 13 gegenüberliegenden Ende einen Verbindungsabschnitt 17. Der Verbindungsabschnitt 17 dient einer Verbindung des Werkzeugs 12 mit einer Eindrehhilfe (nicht gezeigt) wie beispielsweise einer Bohrmaschine oder einem Schraubendrehergriff.

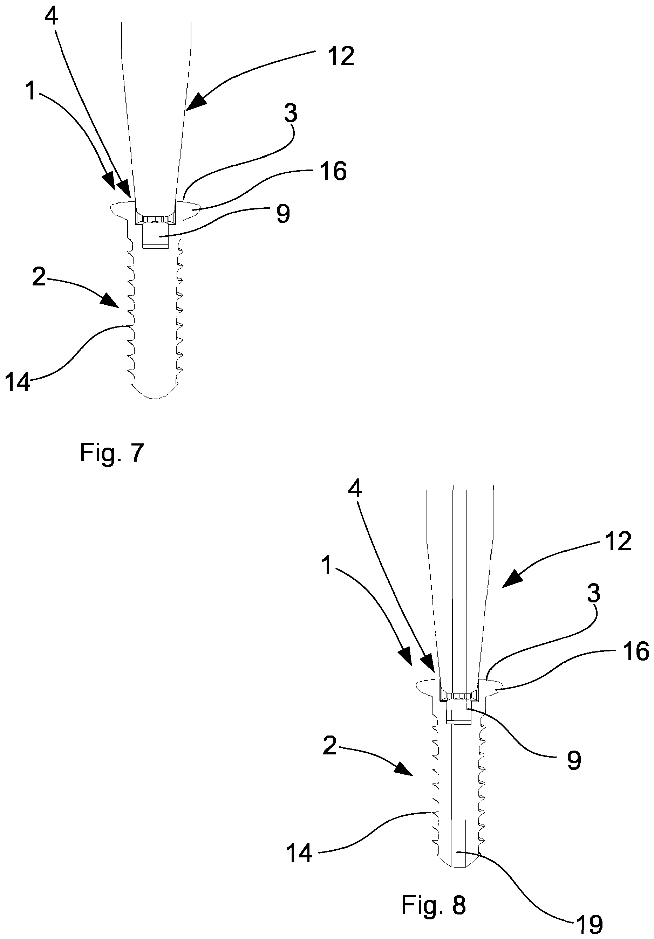

Figur 7 zeigt einen Längsschnitt der Schraube 1 der Figuren 1a, b, nachdem der Pfostenschaft 20 des Einbringpfostens 6 (nicht gezeigt) entfernt wurde und das Werkzeug 12 mit der Gegenkontur 13 in die Ausnehmung 4 eingeführt wurde.

Figur 8 zeigt einen Längsschnitt der Schraube 1 der Figur 3, nachdem der Pfostenschaft 20 des Einbringpfostens 6 (nicht gezeigt) entfernt wurde und das Werkzeug 12 mit der Gegenkontur 13 in die Ausnehmung 4 eingeführt wurde.

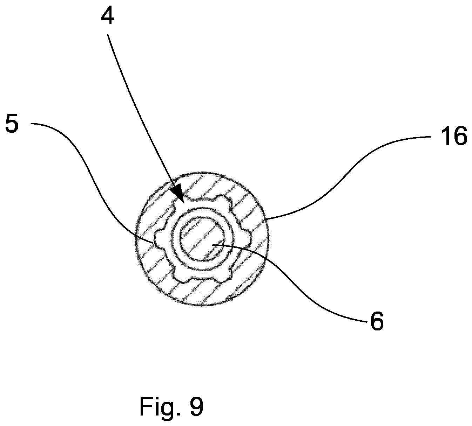

Figur 9 zeigt einen Querschnitt durch einen Schraubenkopf 16 auf Höhe der Sollbruchstelle 7 einer erfindungsgemässen Schraube 1 mit Einbringpfosten 6. Die Ausnehmung 4 des Schraubenkopfes 16 weist eine Kontur 5 in Form eines Innensechsrundes auf. Nach einer Entfernung des Einbringpfostens 6 kann die Schraube 1 über die Kontur 5 mit dem passenden Werkzeug 12 (in Fig. 9 nicht gezeigt) noch weiter eingedreht oder ausgedreht werden.

## Patentansprüche

1. Schraube (1), insbesondere Knochenschraube, mit einem Kopf (16), einem Schaft (2) und einer Spitze (15), wobei die Schraube an einer Kopfseite (3) eine Ausnehmung (4) mit einer Kontur (5) aufweist und in der Ausnehmung (4) mit einem Einbringpfosten (6) versehen ist, der über eine Sollbruchstelle (7) mit der Schraube (1) verbunden ist, wobei eine Gegenkontur (13) eines Werkzeugs (12) nach dem Bruch der Sollbruchstelle mit der Kontur (5) der Ausnehmung (4) in Wirkverbindung bringbar ist,
wobei die Sollbruchstelle (7) innerhalb der Ausnehmung (4) so angeordnet ist, dass nach Entfernung des Einbringpfostens (6) eine Drehmomentübertragung zwischen dem Werkzeug (12) und der Schraube (1) über die Kontur (5) und Gegenkontur (13) möglich ist, wobei der Einbringpfosten (6) einteilig über die Sollbruchstelle (7) mit einer Pfostenspitze (9) versehen ist,
**dadurch gekennzeichnet,**
**dass** die Pfostenspitze (9) eine Kontur für eine Drehmomentübertragung über eine Gegenkontur in der Ausnehmung (4) der Schraube (1) aufweist.

2. Schraube (1) nach Anspruch 1, wobei die Sollbruchstelle (7) versenkt gegenüber der Endfläche (3) der Schraube (1), insbesondere auf dem Niveau der Grundfläche (23) der Kontur (5) der Ausnehmung angeordnet ist.

3. Schraube (1) nach einem der Ansprüche 1 und 2, wobei die Ausnehmung (4) anschliessend an die Kontur (5) eine Öffnung (8), vorzugsweise ein Sackloch, aufweist.

4. Schraube (1) nach einem der Ansprüche 1 bis 3, wobei die Schraube (1) und der Einbringpfosten (6) eine Kanülierung (19, 21) aufweisen.

5. Schraube (1) nach einem der Ansprüche 1 bis 4, wobei die Sollbruchstelle (7) als Materialschwächung in Form einer Einschnürung ausgebildet ist.

6. Schraube (1) nach einem der vorhergehenden Ansprüche, wobei die Sollbruchstelle (7) so ausgelegt ist, dass sie bei einem Drehmoment bricht, das kleiner als das Durchdrehmoment des Knochens ist.

7. Schraube (1) nach Anspruch 3 oder jedem davon abhängigen Anspruch, wobei der Einbringpfosten (6) in die Öffnung (8) eingeschraubt und zusätzlich verpresst ist.

8. Schraube (1) nach Anspruch 3 oder jedem davon abhängigen Anspruch, wobei der Einbringpfosten (6) in die Öffnung (8) der Ausnehmung (4) eingepresst ist.

9. Schraube (1) nach einem der vorhergehenden Ansprüche, wobei die Kontur (5) der Ausnehmung (4) als Sechskant oder Innensechsrund, Vierkant, Phillips, etc. ausgebildet ist.

10. Schraube (1) nach einem der vorhergehenden Ansprüche, wobei die Schraube (1) und der Einbringpfosten aus einem biokompatiblen Material aus der Liste Titan, Titanlegierungen, Stahl, Kunststoff, resorbierbares Metall oder resorbierbarer Kunststoff bestehen.

11. Schraube (1) nach einem der vorhergehenden Ansprüche, wobei der Einbringpfosten (6) eine produktspezifische Kennzeichnung, vorzugsweise eine Lotnr., einen Barcode oder ein Logo, enthält, der nach dem Bruch der Sollbruchstelle (7) ausgelesen und/oder aufbewahrt werden kann.

12. Set, mit wenigstens einer Schraube (1) nach einem der Ansprüche 1-11 und einem Werkzeug (12), das eine zur Kontur (5) der Ausnehmung (4) komplementäre Gegenkontur (13) der Schraube (1) aufweist.

13. Set nach Anspruch 12, welches zusätzlich eine Knochenplatte umfasst.

14. Verfahren zur Herstellung einer Schraube (1) mit einem Einbringpfosten (6), welches die folgenden Schritte umfasst:
- Bereitstellen eines Schraubenkörpers (1) mit einem Kopf, einem Schaft (2) und einer Spitze, wobei der Schraubenkörper (1) eine Ausnehmung (4) mit einer Kontur (5) und anschliessender Öffnung (8), vorzugsweise anschliessendem Sackloch, aufweist.
- Bereitstellung eines Pfostens (6), welcher über eine Sollbruchstelle (7) mit einer Pfostenspitze (9) verbunden ist, wobei die Pfostenspitze (9) eine Kontur für eine Drehmomentübertragung über eine Gegenkontur in der Ausnehmung (4) der Schraube (1) aufweist
- Zusammenfügen des Pfostens (6) und des Schraubenkörpers (1) über die Pfostenspitze (9) und die Öffnung (8).

15. Verfahren nach Anspruch 14, wobei der Pfosten (6) in den Schraubenkörper (1) eingeschraubt und zusätzlich verpresst ist.

16. Verfahren nach Anspruch 14, wobei der Pfosten (6) in den Schraubenkörper (1) eingepresst wird.

## Claims

1. Screw (1), in particular bone screw, having a head (16), a shank (2), and a tip (15), wherein the screw, at a head side (3), has a recess (4) with a contour (5) and is equipped, in the recess (4), with an insertion post (6) which is connected to the screw (1) by way of a predetermined breaking point (7), wherein, after the breaking of the predetermined breaking point, a mating contour (13) of a tool (12) can be brought in operative connection with the contour (5) of the recess (4), wherein the predetermined breaking point (7) is arranged within the recess (4) such that, after removal of the insertion post (6), a transmission of torque between the tool (12) and the screw (1) is possible by way of the contour (5) and mating contour (13)
wherein the insertion post (6) is equipped, integrally by way of the predetermined breaking point (7), with a post tip (9), **characterised in that** the post tip (9) has a contour for a transmission of torque via a mating contour in the recess (4) of the screw (1).

2. Screw (1) according to Claim 1, wherein the predetermined breaking point (7) is arranged so as to be set back relative to the end surface (3) of the screw (1), in particular at the level of the base surface (23) of the contour (5) of the recess.

3. Screw (1) according to either of Claims 1 and 2, wherein the recess (4) has an opening (8), preferably a blind hole, adjoining the contour (5) .

4. Screw (1) according to one of Claims 1 to 3, wherein the screw (1) and the insertion post (6) have a cannulation (19, 21).

5. Screw (1) according to one of Claims 1 to 4, wherein the predetermined breaking point (7) is formed as a material weakening in the form of a constriction.

6. Screw (1) according to one of the preceding claims, wherein the predetermined breaking point (7) is designed so as to break under a torque lower than the breakaway torque of the bone.

7. Screw (1) according to Claim 3 or each claim dependent thereon, wherein the insertion post (6) is screwed and additionally pressed into the opening (8).

8. Screw (1) according to Claim 3 or each claim dependent thereon, wherein the insertion post (6) is pressed into the opening (8) of the recess (4).

9. Screw (1) according to one of the preceding claims, wherein the contour (5) of the recess (4) is in the form of a hexagon or hexalobular socket, square, Phillips etc.

10. Screw (1) according to one of the preceding claims, wherein the screw (1) and the insertion post are composed of a biocompatible material from the list of titanium, titanium alloys, steel, plastic, resorbable metal or resorbable plastic.

11. Screw (1) according to one of the preceding claims, wherein the insertion post (6) comprises a product-specific labelling, preferably a batch number, a barcode or a logo, which can be read off and/or preserved after the breaking of the predetermined breaking point (7).

12. Set having at least one screw (1) according to one of Claims 1-11 and having a tool (12) which has a mating contour (13), which is complementary to the contour (5) of the recess (4), of the screw (1).

13. Set according to Claim 12, which additionally comprises a bone plate.

14. Method for producing a screw (1) having an insertion post (6), comprising the following steps:
- providing a screw body (1) having a head, a shank (2), and a tip, wherein the screw body (1) has a recess (4) with a contour (5) and an adjoining opening (8), preferably adjoining blind hole,
- providing a post (6) which is connected by way of a predetermined breaking point (7) to a post tip (9), wherein the post tip (9) has a contour for a transmission of torque via a mating contour in the recess (4) of the screw (1),
- joining the post (6) and the screw body (1) together by way of the post tip (9) and the opening (8).

15. Method according to Claim 14, wherein the post (6) is screwed and additionally pressed into the screw body (1).

16. Method according to Claim 14, wherein the post (6) is pressed into the screw body (1).

## Revendications

1. Vis (1), en particulier vis à implantation osseuse, comprenant une tête (16), une tige (2) et une pointe (15), la vis comportant sur un côté tête (3) un évidement (4) qui présente un contour (5), et étant pourvue dans l'évidement (4) d'un poteau d'insertion (6) qui est reliée à la vis (1) par un point de rupture défini (7), un contour homologue (13) d'un outil (12) pouvant être relié fonctionnellement au contour (5) de l'évidement (4) après la rupture du point de rupture défini, le point de rupture défini (7) étant disposé à l'intérieur de l'évidement (4) de manière à permettre, après le retrait du poteau d'insertion (6), une transmission de moment entre l'outil (12) et la vis (1) par le biais du contour (5) et du contour homologue (13), le poteau d'insertion (6) étant réalisé d'une pièce avec une pointe de poteau (9) par le biais du point de rupture défini (7), **caractérisée en ce que** le poteau d'insertion (9) présente un contour destiné à la transmission de moment par le biais d'un contour homologue dans l'évidement (4) de la vis (1).

2. Vis (1) selon la revendication 1, le point de rupture défini (7) étant disposé de manière à être enfoncé par rapport à la face d'extrémité (3) de la vis (1), en particulier au niveau de la face de base (23) du contour (5) de l'évidement.

3. Vis (1) selon l'une des revendications 1 et 2, l'évidement (4) comportant, à la suite du contour (5), une ouverture (8), de préférence un trou borgne.

4. Vis (1) selon l'une des revendications 1 à 3, la vis (1) et le poteau d'insertion (6) comportant une canulation (19, 21).

5. Vis (1) selon l'une des revendications 1 à 4, le point de rupture défini (7) étant conçu comme un affaiblissement de matériau se présentant sous la forme d'un étranglement.

6. Vis (1) selon l'une des revendications précédentes, le point de rupture défini (7) étant conçu de manière à se casser à un moment qui est inférieur au moment de patinage de l'os.

7. Vis (1) selon la revendication 3 ou chaque revendication dépendante de celle-ci, le poteau d'insertion (6) étant vissée et étant additionnellement pressé dans l'ouverture (8).

8. Vis (1) selon la revendication 3 ou chaque revendication dépendante de celle-ci, la poteau d'insertion (6) étant pressé dans l'ouverture (8) de l'évidement (4).

9. Vis (1) selon l'une des revendications précédentes, le contour (5) de l'évidement (4) étant conçu de manière à être hexagonal ou rond à six pans creux, carré, de Phillips, etc.

10. Vis (1) selon l'une des revendications précédentes, la vis (1) et la poteau d'insertion étant formées à partir d'un matériau biocompatible de la liste titane, alliages de titane, acier, plastique, métal résorbable ou matière synthétique résorbable.

11. Vis (1) selon l'une des revendications précédentes, le poteau d'insertion (6) contenant une identification spécifique au produit, de préférence un numéro de lot, un code à barres ou un logo, qui peuvent être lus et/ou conservés après la rupture du point de rupture défini (7).

12. Ensemble comprenant au moins une vis (1) selon l'une des revendications 1 à 11, et un outil (12) présentant un contour homologue (13) de la vis (1) complémentaire du contour (5) de l'évidement (4).

13. Ensemble selon la revendication 12, qui comprend en outre une plaque à implantation osseuse.

14. Procédé de fabrication d'une vis (1) comprenant une poteau d'insertion (6), le procédé comprenant les étapes suivantes :
- fournir un corps de vis (1) pourvu d'une tête, d'une tige (2) et d'une pointe, ledit corps de vis (1) comportant un évidement (4) pourvu d'un contour (5) et d'une ouverture adjacente (8), de préférence un trou borgne adjacent,
- fournir un poteau (6) qui est reliée à une pointe de poteau (9) par le biais d'un point de rupture défini (7), la pointe de poteau (9) présentant un contour destiné à la transmission de moment par le biais d'un contour homologue de l'évidement (4) de la vis (1),
- assembler le poteau (6) et le corps de vis (1) par le biais de la pointe de poteau (9) et de l'ouverture (8) .

15. Procédé selon la revendication 14, le poteau (6) étant vissée de vis (1) et étant additionnellement pressé dans le corps.

16. Procédé selon la revendication 14, le poteau (6) étant pressé dans le corps de vis (1).
